**Europäisches Patentamt**

**European Patent Office**

**Office européen des brevets**

(19)

(11) Veröffentlichungsnummer : **0 466 679 A1**

(12) ## EUROPÄISCHE PATENTANMELDUNG

(21) Anmeldenummer : **91890138.0**

(22) Anmeldetag : **02.07.91**

(51) Int. Cl.⁵ : **G01N 15/02**, G01N 33/22, G01N 21/41

(30) Priorität : **12.07.90 AT 1490/90**

(43) Veröffentlichungstag der Anmeldung : **15.01.92 Patentblatt 92/03**

(84) Benannte Vertragsstaaten : **AT DE DK**

(71) Anmelder : **AVL Gesellschaft für Verbrennungskraftmaschinen und Messtechnik mbH.Prof.Dr.Dr.h.c. Hans List Kleiststrasse 48 A-8020 Graz (AT)**

(72) Erfinder : **Wigley, Graham, Dr. Fasslstrasse A-8044 Rabnitz (AT)** Erfinder : **Pitcher, Graham Frank Schröttergasse 1 A-8010 Graz (AT)**

(74) Vertreter : **Krause, Walter, Dr. Dipl.-Ing. et al Postfach 200 Singerstrasse 8 A-1014 Wien (AT)**

(54) **Verfahren zur Bestimmung physikalischer Parameter von Tröpfchen einer zerstäubten Flüssigkeit und Vorrichtung zur Durchführung des Verfahrens.**

(57)    Bei der Verbrennung von Treibstoffnebeln werden die Treibstofftröpfchen einem weiten Temperaturbereich ausgesetzt, wodurch es zu großen Unterschieden in der Dichte und dem Brechungsindex der Tröpfchen kommt. Eine Größenbestimmung der Tröpfchen mit der Phasen-Doppler-Anemometrie nach herkömmlicher Art liefert daher nur ungenaue Werte. Um die Temperatureinflüsse quantitativ zu erfassen, wird vorgeschlagen, daß zur Bestimmung der Temperatur der Tröpfchen bei einem ersten Streuwinkel $\Phi_1$, bei welchem die Phasenverschiebung der Streustrahlung zur Primärstrahlung im wesentlichen nur von der Größe D der Tröpfchen abhängt, die Phasenverschiebung $P_{\Phi_1}(D)$ gemessen und daraus die Größe D der Tröpfchen bestimmt wird, daß simultan bei einem zweiten Streuwinkel $\Phi_2$, bei welchem die Phasenverschiebung der Streustrahlung zur Primärstrahlung von der Größe D der Tröpfchen und deren Brechungsindex n abhängt, die Phasenverschiebung $P_{\Phi_2}(D,n)$ erfaßt und daraus der Brechungsindex n des Tröpfchen bestimmt wird, sowie daß aus der Größe D und dem Brechungsindex n die Dichte und die Temperatur t des Tröpfchens abgeleitet werden.

EP 0 466 679 A1

Jouve, 18, rue Saint-Denis, 75001 PARIS

_Fig. 1_

Die Erfindung betrifft ein Verfahren zur Bestimmung physikalischer Parameter von Tröpfchen einer zerstäubten Flüssigkeit, vorzugsweise eines Treibstoffnebels, mittels Phasen-Doppler-Anemometrie (PDA), sowie eine Vorrichtung zur Durchführung des Verfahrens.

Bei der Phasen-Doppler-Anemometrie werden zur Bestimmung der Größe und der Geschwindigkeit von Tröpfchen einer zerstäubten Flüssigkeit zwei kohärente Anregungsstrahlen gekreuzt, wodurch im Kreuzungsbereich ein bestimmtes Meßvolumen definiert wird. Durch Interferenz der beiden Strahlen entsteht in diesem Meßvolumen ein Muster aus Interferenzstreifen. Über eine Meßeinrichtung, welche die Streustrahlung empfängt, werden Änderungen der Phase bzw. der Frequenz der Streustrahlung erfaßt und daraus Größe und Geschwindigkeit der Tröpfchen abgeleitet.

Die Verbrennung von Treibstoffnebel ist die primäre Energiequelle automobiler aeronautischer und maritimer Transyrortmittel. Weiters spielen sie die Hauptrolle bei der Beheizung von Wohnhäusern und Industrieobjekten, sowie bei der Erzeugung elektrischer Energie. Angesichts ihrer Bedeutung werden die physikalischen Grundlagen der Treibstoffzerstäubung und der nachfolgenden Verbrennungsprozesse jedoch bisher nicht ausreichend verstanden, um eine logische Optimierung der vielfältigen Arten von Verbrennungssystemen zu erlauben.

Durch kürzliche Fortschritte in der Sprühnebeldiagnose ist es möglich geworden, dieses Wissen auch auf die Treibstoffzerstäubung anzuwenden. Die Phasen-Doppler-Anemometrie (PDA), die die gleichzeitige Messung von Größe und Geschwindigkeit einzelner Tröpfchen innerhalb des Nebels erlaubt, wird zusehends auch als Meßmethode zur Untersuchung von Treibstoffnebeln anerkannt. Die Mehrheit der bisher bekanntgewordenen PDA-Anwendungen betraf jedoch ausschließlich sogenannte "kalte" Sprühnebel. Die PDA-Technik wäre an sich jedoch auch auf Treibstoffnebel während bzw. bis zur Verbrennung anwendbar. Da jedoch der Brechungsindex des flüssigen Treibstoffes von der Temperatur abhängig ist, werden nachteiligerweise Messungen der Tröpfchengröße von Änderungen der Tröpfchentemperatur beeinflußt.

Aufgabe der Erfindung ist es, ein Verfahren und eine Vorrichtung zu dessen Durchführung vorzuschlagen, mit weichem die Temperatur der Tröpfchen bzw. die Temperatureinflüsse bei der Messung der Größe von Tröpfchen einer zerstäubten Flüssigkeit, vorzugsweise eines Treibstoffnebels, quantifiziert werden können.

Diese Aufgabe wird erfindungsgemäß dadurch gelöst, daß zur Bestimmung der Temperatur der Tröpfchen bei einem ersten Streuwinkei $\Phi_1$, bei welchem die Phasenverschiebung der Streustrahlung zur Primärstrahlung im wesentlichen nur von der Größe D der Tröpfchen abhängt, die Phasenverschiebung $P_{\Phi1}(D)$ gemessen und daraus die Größe D der Tröpfchen bestimmt wird, daß simultan bei einem zweiten Streuwinkel $\Phi_2$, bei welchem die Phasenverschiebung der Streustrahlung zur Primärstrahlung von der Größe D der Tröpfchen und deren Brechungsindex n abhängt, die Phasenverschiebung $P_{\Phi2}(D,n)$ erfaßt und daraus der Brechungsindex n des Tröpfchen bestimmt wird, sowie daß aus der Größe D und dem Brechungsindex n die Dichte und die Temperatur t des Tröpfchens abgeleitet werden. Eine Streulichtanalyse nach der Phasen-Doppler-Anemometrie hat überraschend gezeigt, daß bei bestimmten Streuwinkeln die Tröpfchengrößenmessungen praktisch unempfindlich sind gegenüber Veränderungen des Brechungsindex. Phasenmessungen bei anderen Streuwinkeln verzerren die Größen-verteilung der Tröpfchemn. Vor allem wird die Größe der Tröpfchen bei höheren Temperaturen überschätzt, falls ein "kalter" Brechungsindex angenommen wird. Durch den Vergleich von für zwei unterschiedliche Streuwinkel gleichzeitig durchgeführten Phasenmessungen, wovon eine vom Brechungsindex unabhängig ist, ist es möglich, den unbekannten Brechungsindex eines Tröpfchens bei höheren Temperaturen zu quantifizierten und daraus dessen Dichte und Temperatur zu berechnen.

In einer Ausgestaltung der Erfindung ist vorgesehen, daß die Phasenverschiebung $P_{\Phi1}(D)$ bezogen auf die Vorwärtsstreurichtung bei einem ersten Streuwinkel $\Phi_1$, im Bereich von 70° und die Phasenverschiebung $P_{\Phi2}(D,n)$ bei einem zweiten StreuwinKel $\Phi_2$ im Bereich von 30° gemessen wird, wobei die Anregungsstrahlung parallel zu den im Meßvolumen vorliegenden Interferenzstreifen (PDA fringe pattern) polarisiert wird.

Eine erfindungsgemäße Vorrichtung zur Durchführung des Verfahrens, mit einer Einrichtung zur Bereitstellung polarisierter, kohänrenter Anregungsstrahlung für ein definiertes Meßvolumen, sowie einer Einrichtung zur Erfassung von vom Meßvolumen abgegebener Streustrahlung (Phasen-Doppler-Anemometrie), wird dadurch realisiert, daß die Meßeinrichtung zumindest zwei, bei unterschiedlichen Streuwinkeln $\Phi_1$ und $\Phi_2$ messende Detektionssysteme aufweist, daß eines der Detektionssysteme auf Streustrahlung im Bereich von 70° und das andere auf Streustrahlung im Bereich von 30°, bezogen auf die Vorwärtsstreurichtung, ausgerichtet ist, sowie daß die Detektionssysteme simultan ein der jeweiligen Phasenverschiebung bei den beiden Streuwinkeln $\Phi_1$ und $\Phi_2$ proportionales Signal erzeugen, welche Signaie einer Auswerteeinheit zuführbar sind.

Die folgrenden Ausführungen gelten für Treibstofftröpfchen, sollen die Erfindung jedoch nicht darauf einschränken. Die Untersuchungen wurden für einem bestimmten Bereich der Tröpfchengröße und des Brechungsindex durchgeführt.

Die Treibstoffdichte - und folglich der Brechungsindex - wird direkt vom örtlichen Druck und der örtlichen Temperatur beeinflußt. In einem Verbrennungssystem variieren Druck und Temperatur sowohl zeitlich als auch örtlich und hängen vor allem vom Mengendurchsatz des Treibstoffs, der Oberflächengröße, der Verdampfungsrate und dem örtlichen Verbrennungszustand ab.

In einer ersten Annäherung an das Problem wird eine Schätzung des maximalen Temperaturbereichs vorgenommen, dem der Treibstoff ausgesetzt sein kann. Das ermöglicht die Bestimmung der Bereichsgrenzen für den Brechungsindex n. Die Zimmertemperatur bzw. die Eintrittstemperatur des Treibstoffs in das Verbrennungssystem, wie z.B. die Temperatur an der Spitze der Ein spritzdüse, kann ais niedrigste Treibstofftemperatur angenommen werden. Die höchste Temperatur des Treibstoffs in seinem flüssigen Zustand kann für Hochdruckverbrennungssysteme mit seiner kritischen Temperatur oder für Niederdruckverbrennungssysteme mit seinem Siedepunkt gleichgesetzt werden.

Der maximal mögliche Bereich von Brechungsindizes liegt somit zwischen einem schweren Treibstoff bei Zimmertemperatur und einem leichten Treibstoff bei seiner kritischen Temperatur $T_k$. Dieselöl und Ethanol werden stellvertretend für einen schweren bzw. leichten Treibstoff für die Untersuchung herangezogen.

Daten über Brechungsindizes bei Zimmertemperatur sind kaum verfügbar, jene über höhere Temperaturen noch weniger. Gelegentlich sind jedoch Daten über die Veränderung der Treibstoffdichte mit der Temperatur verfügbar und diese können in Verbindung mit der Eykman-Gleichung zur Berechnung des Brechungsindex verwendet werden. Die Veränderung des Brechungsindex n von Dieselöl und Ethanol mit der Dichte d und der Temperatur T ist in Tabelle 1 zu sehen.

## TABELLE 1

Eykman-Gleichung: $(n^2-1)/(n+0.4) = const . d$

|  | T °K | d kg/m₃ | n |
|---|---|---|---|
| Dieselöl: | 293 | 840 | 1,45 |
|  | 423 | 785 | 1,43 |
| Ethanol: | 293 | 788 | 1,36 |
|  | 348 | 723 | 1,33 |
|  | 442 $T_s$ | 597 | 1,27 |
|  | 503 $T_K$ | 490 | 1,22 |

Die Untersuchung von Dieselöl erweist sich ais schwierig, da es aus verschiedenen Komponenten besteht, die - als Folge ihrer unterschiedlichen Flüchtigkeit - große Unterschiede in ihren physikalischen Eigenschaften aufweisen. Folglich liegt der Siedepunkt $T_s$ z.B. in einem Bereich von 425 bis 695°K.

Es hat sich gezeigt, daß ein lineares Verhältnis zwischen Phase und Tröpfchengröße im al allgemeinen dann anzutreffen ist, wenn nur ein Streumechanismus, z.B. Reflektion oder Brechung vorherrscht. Eine theoretische Analyse der Streustrahlung wird nun durchgeführt, um die beschriebenen, vorteilhaften Streugeometrien zu verifizieren, wobei vorerst Tröpfchen von 5 Mikrometer Durchmesser mit Brechungsindizes von 1,22 und 1,45 angenommen werden.

Die numerischen Berechnungen basieren auf einem einfachen Modell für die Streustrahlung von zwei gekreuzten, kohärenten und ebenen Wellen, die den Meßraum für die PDA darstellen. Das von jeder ebenen Welle gestreute Licht wird jeweils mitteis geometrischer Optik, d.h. Beugung, Reflektion oder Brechung, oder der Mie-Theorie, deren Werte den gemessenen Werten entsprechen, berechnet. Für die Berechnung wird angenommen, daß die Tröpfchen homogen und kugelförmig sind, mit einem beliebig komplexen Brechungsindex für lichtabsorbierende Teilchen. Die beiden gestreuten Wellen werden kohärent addiert und numerisch integriert. Für jede vorgebbare optische Geometrie kann die Polarisation, die Amplitude, und die Phase der PDA-Signale bestimmt werden.

Im folgenden wird die Erfindung anhand von Zeichnungen näher erläutert. Es zeigen:

Fig. 1 eine Vorrichtung nach der Erfindung in schematischer Darstellung,

Fig. 2 die Entstehung der unterschiedlichen Streustrahlungsanteiie an einem Tröpfchen, sowie die

Fig. 3 bis 15 Diagramme von Meßwerten und theoretischen Vergleichswerten.

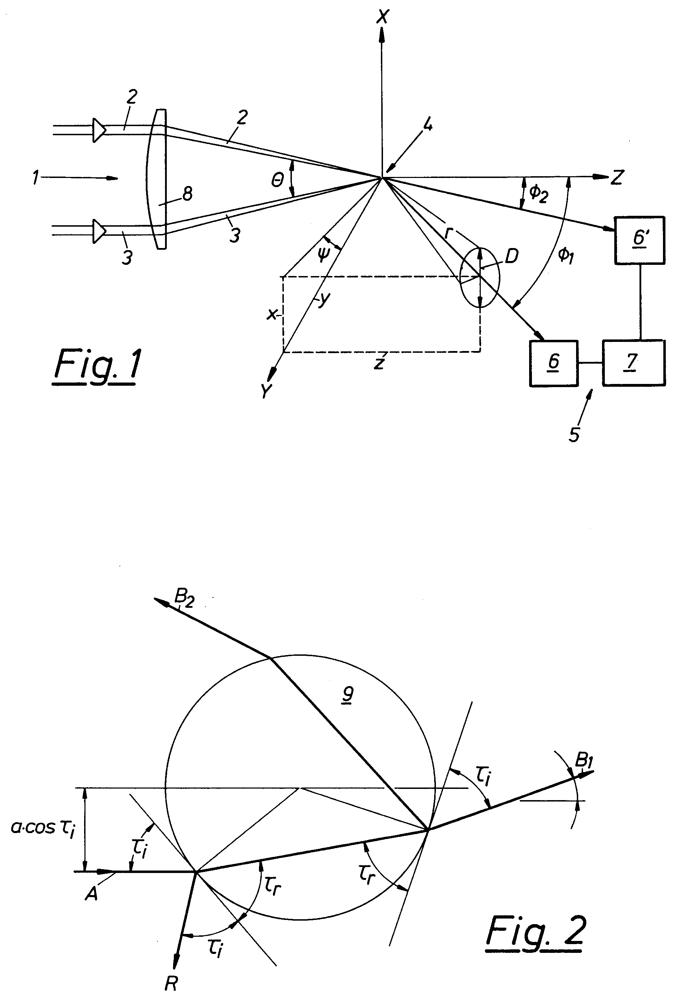

Die in Fig. 1 schematisch dargesteilte Vorrichtung weist eine nicht weiter präzisierte Einrichtung 1 zur Bereitstellung polarisierter, kohärenter Anregungsstrahlung auf (z.B. ein Laser), deren beiden Strahlen 2 und 3 im Kreuzungsbereich ein Meßvolumen 4 definieren, in weiches der Ursprung eines Koordinatensystems mit den Achsen x, y und z gelegt wurde. Die Meßeinrichtung 5 weist zwei, bei unterschiedlichen Streuwinkeln $\Phi_1$,

und $\Phi_2$ simultan messende Detektionssysteme 6, 6′ auf. Die Detektionssysteme sind über Signalleitungen mit einer Auswerteeinheit 7 verbunden. Beispielsweise wird die Anregungsstrahlung von einem Laser mit einer Wellenlänge von 0,5145 µm und einer Leistung von 0,2 Watt bereitgestellt. Der Strahlabstand zwischen den beiden Strahlen 2 und 3 beträgt 50 mm und die Brennweite der Linse 8 450 mm. Der von den Strahlen 1 und 2 eingeschlossene Winkel θ beträgt 7,36°. Der Durchmesser des Meßvolumen beträgt 60 µm, der Abstand der Interferenzstreifen im Meßvolumen 4,64 µm. Die Eingangsbiende der Detektorsysteme 6, 6′ weist einen Durchmesser von 40 (20) mm auf die Distanz r beträgt 310 mm. Der Höhenwinkei psi für die Messungen liegt bei 3,7 bis 4,5°.

In Fig. 2 ist ein Tröpfchen 9 eines Treibstoffnebeis dargestellt, wobei im Hinblick auf eine Einstrahlrichtung A die Teilstrahlen für Reflexionen R, Brechung 1. Ordnung $B_1$ und Brechung 2. Ordnung $B_2$ dargestellt sind.

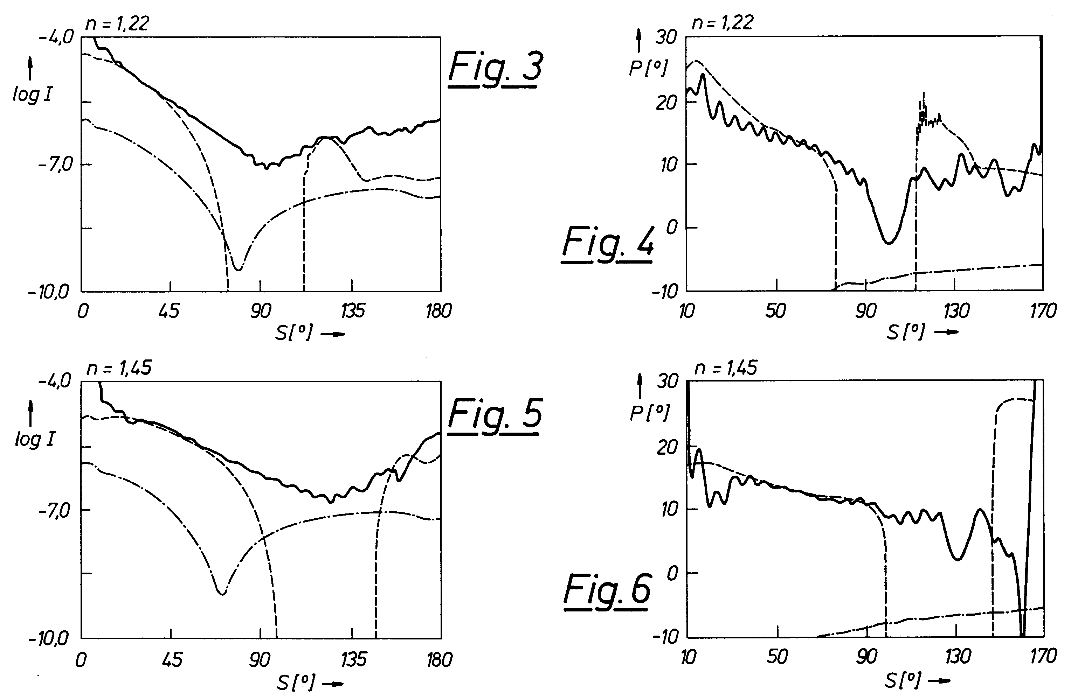

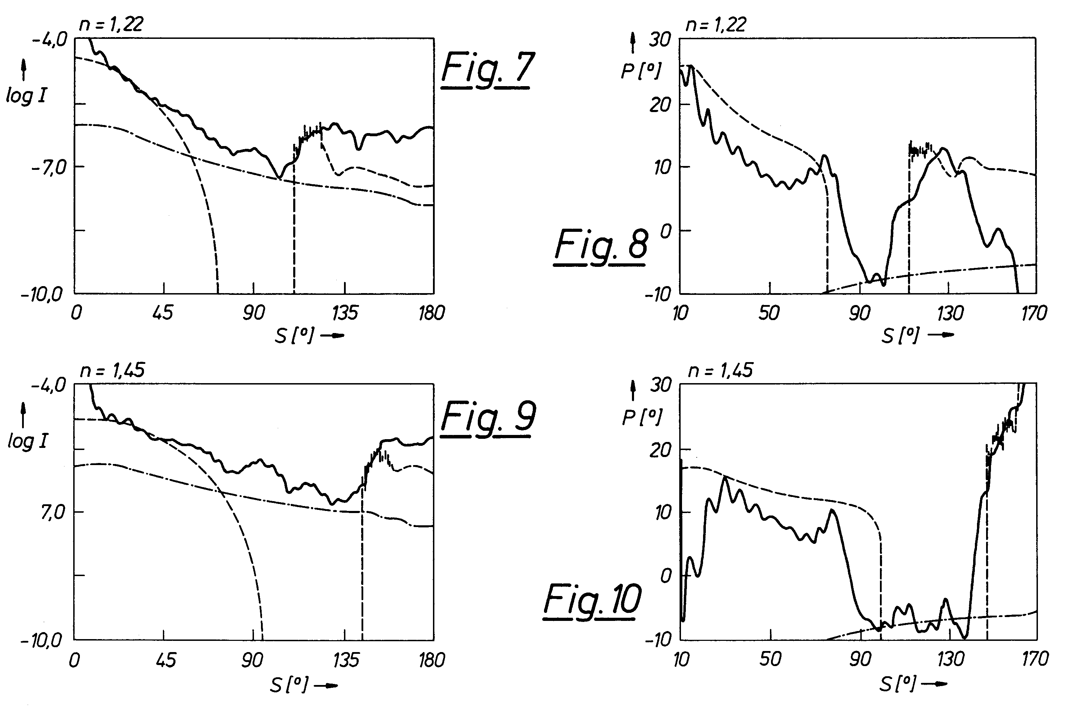

Die Messungen bzw. Berechnungen der Intensität und des Phasenwinkels des Streulichtes in Abhängigkeit des Streuwinkeis, dargestellt in den Diagrammen 3 bis 10 wurden für zwei orthogonale Ausrichtungen des einfallenden, linear polarisierten Laserlichtes durchgeführt und zwar in den Fig. 3 bis 6 für eine Polarisation parailei zu y-z-Ebene und in den Fig. 7 bis 10 für eine Polarisation normal auf die y-z-Ebene (siehe Fig. 1).

Auf der Ordinate ist jeweils die Intensität I der Strahlung (logarithmisch dargestellt) bzw. der Phasenwinkel P in Grad und auf der Abszisse der Streuwinkei S in Grad aufgetragen, wobei 0° in die Vorwärtsstreurichtung und 180° in die Rückstreurichtung weist.

Die der Mie-Theorie entsprechenden Kurven sind jeweils fett dargestellt, die Kurven nach der Theorie der geometrischen Optik sind für den reflektierten Strahlungsanteil strichpunktiert und für den gebrochenen Strahlungsanteil (1. und 2. Ordnung) strichliert dargestellt. In allen Diagrammen zeigt der gebrochene Lichtanteil eine Lücke zwischen maximalem Winkel für die Brechung 1. Ordnung und minimalem Winkel für die Brechung 2. Ordnung. Diagramme mit den Grenzwerten für den Brechungsindex n = 1,22 und n = 1,45 werden jeweils gegenübergestellt. Die Intensität der reflektierten Strahlung, für Anregungsstrahlung die parallel zur y-z-Ebene polarisiert ist, zeigt ein Minimum, welches bei einer Abnahme des Brechungsindex von 1,45 zu 1,22 von einem Streuwinkel von 70° zu einem von 80° wandert, wobei der maximale Winkel für Streustrahlung 1. Ordnung von ca. 95° auf ca. 70° abnimmt (Fig. 3 und 5).

Im Falle der Polarisation der einfallenden Anregungsstrahlung parallel zur y-z-Ebene, d.h. parallel zu den Interferenzstreifen im Meßvolumen 4 (Fig. 3 bis 6), zeigt nur die gebrochene Strahlung 1. Ordnung Signalstärken und Phasenwinkel, die mit der Mie-Theorie für die zwei Brechungsindizes von 1,22 und 1,45 vergleichbar sind. Der Übereinstimmungsbereich, bzw. das Meßfenster liegt für den höheren Brechungsindex zwischen ca. 30 und 70°. Für niedrige Brechungsindizes im Bereich von n=1,22 zeigt sich in Fig. 3 ein Übereinstimmungsbereich zwischen ca. 20 und 45°, während die Phasenkurven (Fig. 4) ein Fernster von ca. 45° bis 75° aufweisen.

Im Falle der Polarisation der einfallenden Strahlung senkrecht zur y-z-Ebene (Fig. 7 bis 10), zeigt nur das reflektierte Licht (strichpunktierte Linie) Signalstärken und Phasenwinkel, die mit der Mie-Theorie übereinstimmen. Der niedrigste gemeinsame Streuwinkel S für das Meßfenster beträgt ca. 95° für beide Brechungsindizes. Der obere Streuwinkel, der durch das Auftreten von Brechung 2. Ordnung bestimmt ist, sinkt jedoch dramatisch von ca. 135° auf 105°, wenn der Brechungsindex von 1,45 auf 1,22 fällt, sodaß nur ein Meßfenster zwischen 95 und 105° existiert.

Da nur für jene Streuwinkei eine lineare Beziehung zwischen Tröpfchengröße und Phase zu erwartem ist, bei welchen die Werte der Mie-Theorie mit den Werten der gebrochenen oder reflektierten Streulichtanteiie zusammenfallen, ergeben sich folgende zwei Meßgeometrien.

a) Die Anregungsstrahlung wird senkrecht zu den im Meßvolumen vorliegenden Interferenzstreifen polarisiert, wobei reflektierte Strahlung bei einem Streuwinkei zwische 95° und 105° detektiert wird.

b) Die Anregungsstrahlung wird parallel zu den im Meßvolumen vorliegenden Interferenzstreifen polarisiert, wobei gebrochene Strahlung 1. Ordnung bei einem Streuwinkel zwischen 30° und 70° detektiert wird. Weitere Untersuchungen haben jedoch gezeigt, daß in der Meßgeometrie a) die Phasenverschiebung zwar vom Brechungsindex n unabhängig ist, jedoch eine nichtlineare Beziehung zwischen Tröpfchengröße und Phase für Tröpfchen mit einem Durchmesser kleiner 30 µm besteht. Diese Meßgeometrie kann also nur mit Einschränkungen verwendet werden.

In der Meßgeometrie b) hat eine Untersuchung der Beziehung zwischen Phase und Tröpfchengröße für Brechungsindices zwischen 1,22 und 1,45 gezeigt, daß nur für Streuwinkei $\Phi_1$, im Bereich von 70° eine vom Brechungsindex n unabhängige Phasenverschiebung $P_{\Phi_1}(D)$ zu erwarten ist und die Phasenverschiebung $P_{\Phi_2}(D,n)$ bei einem streuwinkel $\Phi_2$ im Bereich von 30° eine starke Abhängigkeit vom Brechungsindex n aufweist. Eine einfache Erklärung dafür ist in der Länge des Lichtweges innerhalb der Tröpfchen zu finden, weiche bei einem Streuwinkei von 30° länger ist als bei einem von 70°.

Eine erfindungsgemäße Vorrichtung muß deshalb zwei PDA-Detektionssysteme aufweisen, eines im Bereich von 70° und eines im Bereich von 30°, jeweils bezogen auf die Vorwärtsstreurichtung.

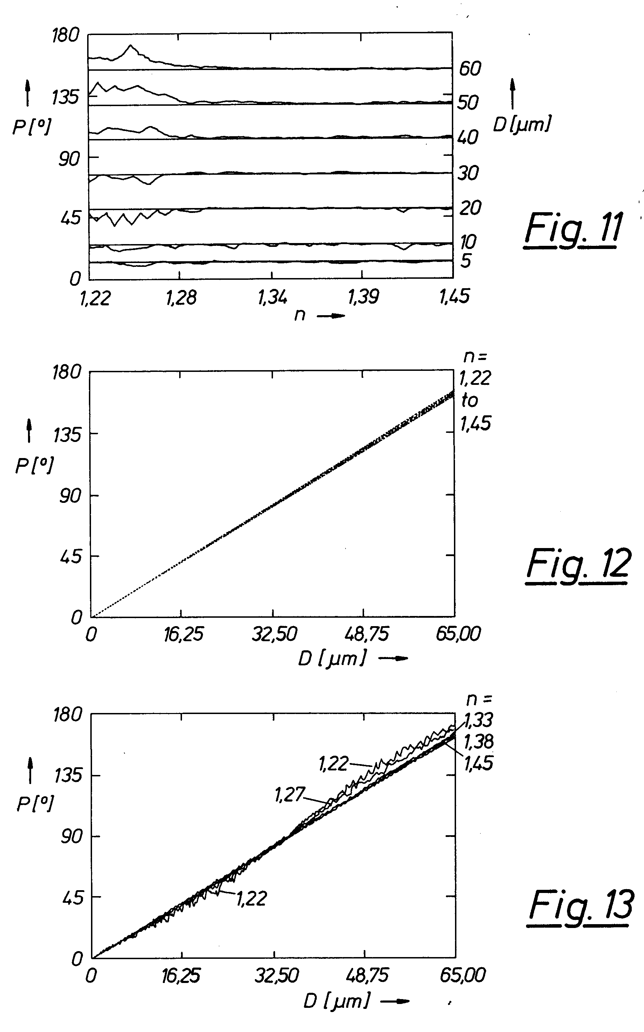

Für eine 70° Streugeometrie werden in den Diagrammen der Fig. 11 bis 13 Meßwerte und theoretische Werte gegenübergestellt.

In Fig. 11 werden für festgelegte Tröpfchenklassen von 5, 10, 20, 30, 40, 50 und 60 μm Durchmesser Veränderungen des Phasenwinkels P für unterschiedliche Brechungsindizes (n=1,22 bis 1,45) erfaßt.

Aus Fig. 11 ist ersichtlich, daß die Beziehung zwischen Phasenwinkel und Brechungsindex praktisch für alle Tröpfchenklassen linear und unempfindlich gegenüber Änderungen im Brechungsindex n verläuft, sobald der Brechungsindex n größer ais 1,27 ist (die durchgezogenen Linien entsprechen den theoretischen Werten der geometrischen Optik).

Für die gewählte 70°-Geometrie könnte z.B. ein einziger Kalibrierfaktor von 5,01 Grad Phasenwinkel pro Mikrometer Durchmesser der Tröpfchen gewählt werden.

In den Diagrammen der Fig. 12 und 13 ist jeweils für die 70° Streugeometrie auf der Abszisse der Tröpfchendurchmesser D in μm und auf der Ordinate der Phasenwinkel P in Grad aufgetragen. Man sieht, daß die Kurven für die gebrochene Strahlung 1. Ordnung für die Brechungsindizes 1,22 bis 1,45 beinahe zusammenfallen (Fig. 12). Gleiches gilt für die Werte nach der Mie-Theorie, welche gemessenen Werten entsprechen.

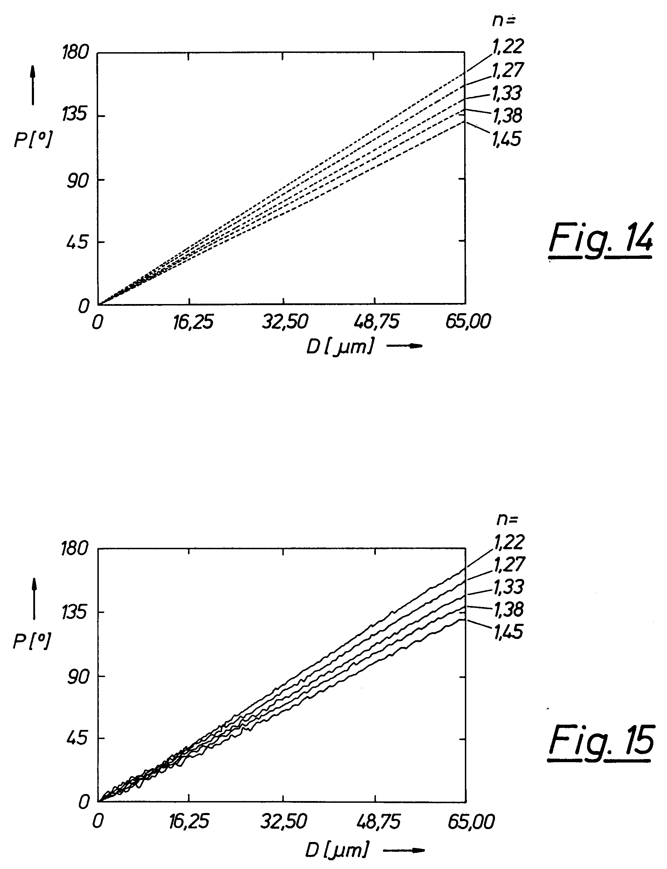

In den Diagrammen Fig. 14 und 15 ist jeweils für eine 30° Streugeometrie die Abhängigkeit des Phasenwinkels von der Tröpfchengröße aufgetragen. Dabei läßt sich sowohl für die gebrochene Strahlung 1. Ordnung ais auch für die Werte nach der Mie-Theorie eine Abhängigkeit von Brechungsindex n entnehmen. Damit kann im Bereich zwischen n=1,27 und 1,45 der Brechungsindex mit einer Genauigkeit von 1 % bestimmt werden, und daraus die Temperatur der Tröpfchen abgeleitet werden.

## Patentansprüche

1. Verfafren zur Bestimmung physikalischer Parameter von Tröpfchen einer zerstäubten Flüssigkeit, vorzugsweise eines Treibstoffnebels, mittels Phasen-Doppler-Anemometrie (PDA), **dadurch gekennzeichnet**, daß zur Bestimmung der Temperatur der Tröpfchen bei einem ersten Streuwinkel $\Phi_1$, bei welchem die Phasenverschiebung der Streustrahlung zur Primärstrahlung im wesentlichen nur von der Größe D der Tröpfchen abfängt, die Phasenverschiebung $P_{\Phi 1}$,(D) gemessen und daraus die Größe D der Tröpfchen bestimmt wird, daß simultan bei einem zweiten Streuwinkel $\Phi_2$, bei welchem die Phasenverschiebung der Streustrahlung zur Primärstrahlung von der Größe D der Tröpfchen und deren Brechungsindex n abfängt, die Phasenverschiebung $P_{\Phi 2}$(D,n) erfaßt und daraus der brechungsindex n des Tröpfchen bestimmt wird, sowie daß aus der Größe D und dem Brechungsindex n die Dichte und die Temperatur t des Tröpfchens abgeleitet werden.

2. Verfahren nach Anspruch 1, **dadurch gekennzeichnet,** daß die Phasenverschiebung $P_{\Phi 1}$(D) bezogen auf die Vorwärtsstreurichtung bei einem ersten Streuwinkel $\Phi_1$ im Bereich von 70° und die Phasenverschiebung $P_{\Phi 2}$(D,n) bei einem zweiten Streuwinkel $\Phi_2$ im Bereich von 30° gemessen wird, wobei die Anregungsstraflung parallel zu den im Meßvolumen vorliegenden Interferenzstreifen (PDA fringe pattern) polarisiert wird.

3. Vorrichtung zur bestimmung physikalischer Parameter von Tröpfchen einer zerstäubten Flüssigkeit, vorzugsweise eines Treibstoffnebels, mittels Phasen-Doppler-Aneometrie (PDA), mit einer Einrichtung (1) zur Bereitstellung polarisierter, kohärenter Anregungsstrahlung für ein definiertes Meßvolumen (4), sowie einer Einrichtung (5) zur Erfassung von vom Meßvolumen (4) abgegebener Streustrahlung, dadurch gekennzeichnet, daß die Meßeinrichtung (5) zumindest zwei, bei unterschiedlichen Streuwinkeln $\Phi_1$ und $\Phi_2$ messende Detektionssysteme (6, 6′) aufweist, daß eines der Detektionssysteme (6) auf Streustrahlung im Bereich von 70° und das andere (6′) auf Streustrahlung im bereich von 30°, bezogen auf die Vorwärtsstreurichtung (Z), ausgerichtet ist, sowie daß die Detektionssysteme (6, 6′) simultan ein der jeweiligen Phasenverschiebung bei den beiden Streuwinkeln $\Phi_1$ und $\Phi_2$ proportionales Signal erzeugen, welche Signale einer Auswerteeinheit (7) zuführbar sind.

_Fig.1_

_Fig.2_

Fig. 3

Fig. 4

Fig. 5

Fig. 6

EP 0 466 679 A1

Fig. 7

Fig. 8

Fig. 9

Fig. 10

9

_Fig. 11_

_Fig. 12_

_Fig. 13_

_Fig. 14_

_Fig. 15_

Europäisches
Patentamt

# EUROPÄISCHER RECHERCHENBERICHT

Nummer der Anmeldung

EP    91 89 0138
Seite 1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | APPLIED OPTICS<br>vol. 20, no. 10, 15 Mai 1981, NEW-YORK, USA<br>Seiten 1803 - 1814; A.ASHKIN ET AL.:<br>"Observation of optical resonances of dielectric spheres by light scattering"<br>* Zusammenfassung *<br>* Seite 1804, Spalte 2, Zeilen 20 - 49 *<br>* Seite 1806, Spalte 2, Zeilen 1 - 7 *<br>* Seite 1813, Spalte 2, Zeile 5 - Seite 1814, Spalte 1, Zeile 22 *<br>--- | 1-3 | G01N15/02<br>G01N33/22<br>G01N21/41 |
| A | APPLIED OPTICS.<br>vol. 28, no. 3, 01 Februar 1989, NEW YORK US<br>Seiten 530 - 536; M.R. ZACHARIAH ET AL.:<br>"Dynamic light scattering and angular dissymetry..."<br>* Zusammenfassung *<br>* Seite 530 : Introduction *<br>* Seiten 533 - 535 : Results and Discussion *<br>* Seite 536 : Table 1 *<br>--- | 1-3 | |
| A | SOVIET INVENTIONS ILLUSTRATED. Section Ch.:Chemical, Week K01, 16 Feb. 1983. Derwent Pub. Ltd., London GB. * Class J, no. 02051 *<br>& SU-A-911-232 (EXPER METEOR.), 26 June 1980<br>--- | 1-3 | RECHERCHIERTE SACHGEBIETE (Int. Cl.5)<br><br>G01N<br>G01P |
| A | SOVIET INVENTIONS ILLUSTRATED. Section El.:Electrical, Week 8818, 5 May 1988. Derwent Pub. Ltd., London, GB. * Class S, no. 88-125498/18 *<br>& SU-A-1343-303 (GRITSENKO A P) , 7 Octob. 1987<br>--- | 1-3 | |
| A | SOVIET INVENTIONS ILLUSTRATED, Section El.: Electrical, Week K24, 27 Jul.1983, Derwent Pub. Ltd. London GB. * Class S, J 0790 *<br>& SU-A-949-422 (AS BELO PHYS INST) 07 Aug. 1982<br>---<br>-/-- | 1-3 | |

Der vorliegende Recherchenbericht wurde für alle Patentansprüche erstellt

| Recherchenort | Abschlußdatum der Recherche | Prüfer |
|---|---|---|
| BERLIN | 28 OKTOBER 1991 | MOUTARD P. |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus andern Gründen angeführtes Dokument
................................................................
& : Mitglied der gleichen Patentfamilie, übereinstimmendes
    Dokument

EPO FORM 1503 03.82 (P0403)

Europäisches
Patentamt

# EUROPÄISCHER RECHERCHENBERICHT

Nummer der Anmeldung

EP    91 89 0138
Seite 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| P,A | PATENT ABSTRACTS OF JAPAN vol. 14, no. 430 (P-1106)(4373) 14 September 1990, & JP-A-02 168138 (TORAY IND. INC.) 28 Juni 1990, * das ganze Dokument * ----- | 1-3 | |
| | | | **RECHERCHIERTE SACHGEBIETE (Int. Cl.5 )** |

Der vorliegende Recherchenbericht wurde für alle Patentansprüche erstellt

| Recherchenort | Abschlußdatum der Recherche | Prüfer |
|---|---|---|
| BERLIN | 28 OKTOBER 1991 | MOUTARD P. |

EPO FORM 1503 03.82 (P0403)